(19) Europäisches Patentamt
European Patent Office
Office européen des brevets

(11) Publication number: 0 125 909 B1

(12) **EUROPEAN PATENT SPECIFICATION**

(45) Date of publication of patent specification: 27.07.88

(51) Int. Cl.⁴: C 07 C 172/00, A 61 K 31/59

(21) Application number: 84303226.9

(22) Date of filing: 11.05.84

# (54) Ring A- and triene-modified vitamin D compounds.

(30) Priority: 11.05.83 US 493537

(43) Date of publication of application: 21.11.84 Bulletin 84/47

(45) Publication of the grant of the patent: 27.07.88 Bulletin 88/30

(84) Designated Contracting States: BE CH DE FR GB IT LI NL SE

(56) References cited:
US-A-4 224 231
US-A-4 229 359

JOURNAL OF ORGANIC CHEMISTRY, vol. 48, no. 21, October 21, 1983, pages 3819-3820; Washington D.C., US H. E. PAAREN et al.: "Electrophilic addition of OsO4 to 25-hydroxycholecaliferol and its 3,5-cyclo derivative."

(73) Proprietor: WISCONSIN ALUMNI RESEARCH FOUNDATION
614 North Walnut Street
Madison, WI 53705 (US)

(72) Inventor: DeLuca, Hector F.
5130 Minocqua Crescent
Madison, WI 53705 (US)
Inventor: Schnoes, Heinrich K.
1806 Summit Avenue
Madison, WI 53705 (US)
Inventor: Paaren, Herbert E.
4408 Smith Drive
Deerfield Wisconsin 53531 (US)
Inventor: Smith, Connie M.
2918 Post Road
Madison, WI 53713 (US)

(74) Representative: Ellis-Jones, Patrick George Armine et al
J. A. KEMP & CO.
14 South Square Gray's Inn
London WC1R 5EU (GB)

EP 0 125 909 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to vitamin D compounds in which ring A and/or the triene chromophore is modified.

This discovery of biologically active vitamin D metabolites characterized by hydroxy substitution at carbon 1 and/or carbon 25 of the vitamin D skeleton has led to much activity in the area of the chemical synthesis of vitamin D compounds. Much of that activity has been concerned with the preparation of the biological metabolites and of close structural analogs of these compounds. Synthetic approaches and results are summarized in a number of recent reviews (e.g. DeLuca *et al.,* Topics in Current Chemistry, vol. 83, p. 1 (1979); Yakhimovich, Russian Chem. Revs. *49,* 371 (1980); Lythgoe, Chem. Soc. Rev. *9,* 449 (1980)).

The present invention relates to the preparation of a new class of vitamin D compounds characterized by modified ring A and triene structures. More specifically, the invention cencerns 7,8-dihydroxy-, 10,19-dihydroxy- and the 10-oxo-19-nor-analogs of 25-hydroxyvitamin $D_3$ (25-hydroxycholecalciferol) and the hydroxy-protected derivatives thereof, the structures of which are given below.

The term "hydroxy-protecting group", as used herein means any group which protects the hydroxy group from attack such as acyl, alkylsilyl, tetrahydropyranyl and methoxymethyl. The term "acyl" signifies an aliphatic acyl group, generally of from 1 to 5 carbons, in all its isomeric forms, or an aromatic acyl group, such as benzoyl, or halogen-, alkyl- or nitro-substituted benzoyl. The term "alkyl" signifies an aliphatic hydrocarbon group generally of 1 to 5 carbons in all its isomeric forms, e.g. methyl, ethyl, propyl and tert.-butyl.

The compounds of this invention can be prepared as follows:

Treatment of a 3,5-cyclovitamin derivative of 25-hydroxyvitamin $D_3$, (e.g. 25-hydroxy-3,5-cyclovitamin $D_3$ 6-methyl ether, a known compound which can be prepared from 25-hydroxycholecalciferol as described in U.S. Patent 4,195,027) with osmium tetroxide in pyridine solution gives the corresponding 10,19-dihydroxy analog, represented by structure *1* below, where $R_1$, $R_2$ and $R_3$ represent hydrogen and X is an alkyl group, e.g. methyl.

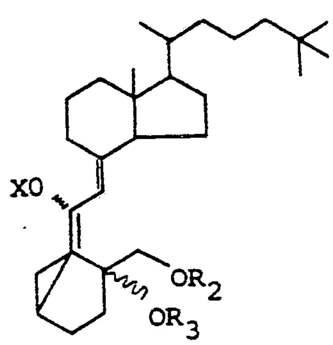

1

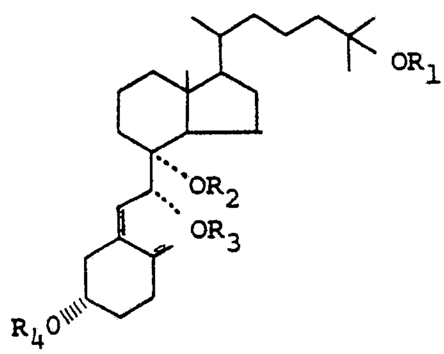

2

The regiospecificity of this reaction, i.e. the specific hydroxylation of the 10(19)-double bond, in preference to the alternative 7,8-double bond, is remarkable and unexpected, especially so since treatment of 25-hydroxycholecalciferol 3-acylate itself, with osmium tetroxide under similar conditions leads to the 7,8-dihydroxy analog, structure *2* above ($R_1$, $R_2$, $R_3$ = H and $R_4$ = Acyl) where again the absence of hydroxylation at any of the other available double bonds (C-5,6 and C-10(19)) is a very notable feature. Compounds of formula *1* and *2* are novel. Using the above general procedure, the osmomylation of other 25-hydroxy-cyclovitamin D analogs, e.g. other 25-hydroxy-3,5-cyclovitamin $D_3$ 6-alkyl ethers, where the alkyl group is, for example, ethyl, propyl, isopropyl or butyl, provides the corresponding 10,19-dihydroxy compounds of general structure *1*, above, where $R_1$, $R_2$ and $R_3$ represent hydrogen and X is an alkyl group corresponding to that at the 6 position in the starting material; these 6-0-alkyl analogs are also suitable substrates for the subsequent reaction process described below.

From the hydroxy compounds thus prepared, the corresponding hydroxy-protected derivatives can be obtained by conventional hydroxy-protecting procedures known in the art (e.g. conventional acylation or alkylsilylation procedures) to obtain the corresponding partially or fully hydroxy-protected derivatives represented by structures *1* or *2* above, where one or more of $R_1$, $R_2$, $R_3$ and $R_4$ represent a hydroxy-protecting group.

Solvolysis of compound *1* above (where $R_1$, $R_2$, $R_3$ = H; X = alkyl) in a medium including an organic acid according to the general procedure of US—A—4,195,027 and US—A—4,260,549, provides the novel 5,6-*cis* and 5,6-*trans* vitamin D-triols, represented by structures *3* and *4* ($R_1$, $R_2$, $R_3$ = H) respectively, wherein $R_4$ is an acyl group corresponding to the acyl part of the acid used in the solvolysis medium.

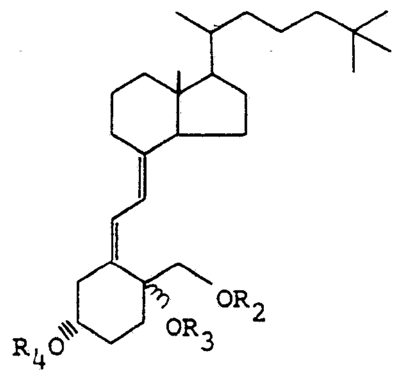

3

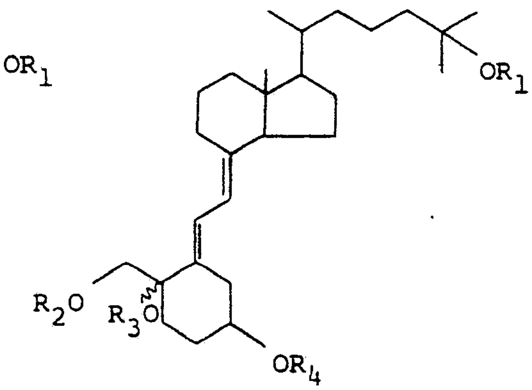

4

The *cis* and *trans*-isomers resulting from solvolysis are conveniently separated (e.g. by chromatography) at this stage. They can be deacylated by conventional base hydrolysis or hydride reduction to the free hydroxy compounds, represented by *3* and *4* above, where $R_1$, $R_2$, $R_3$ and $R_4$ are H. Conventional hydroxy-protecting procedures, e.g. acylation or alkylsilylation, applied to the free hydroxy compounds thus obtained, or to the corresponding 3-O-acyl derivatives, provide, depending on the choice of reagent and conditions, the corresponding partially or completely hydroxy-protected derivatives, i.e. compounds of structure *3* and *4* above, where each $R_1$, $R_2$, $R_3$ and $R_4$ is independently hydrogen, acyl or alkylsilyl or other hydroxy protecting group.

Cleavage of the vicinal 10,19-diol of compounds *3* or *4* affords novel 10-oxo-vitamin analogs. Thus, reaction of compound *3* ($R_1$, $R_2$, $R_3$, $R_4$ = H) with sodium metaperiodate in a suitable solvent gives the 10-oxo-vitamin analog of structure *5*, below ($R_1$, $R_4$ = H). Similar treatment of the 5,6-trans-compound *4* ($R_1$, $R_2$, $R_3$, $R_4$ = H) provides the corresponding 10-oxo-analog *6* ($R_1$, $R_4$ = H). The 3-O-monoacyl derivatives of structure *3* or *4* (as obtained, for example, from the solvolysis reaction) are equally suitable substrates for the periodate cleavage process, and yield the corresponding 3-O-acyl-10-oxo compounds of structures *5* and *6*, respectively, where $R_1$ = H and $R_4$ = acyl, which can be converted to the 3-hydroxy-compounds by standard alkaline hydrolysis, or which, if desired, can be further hydroxy-protected at the C-25-hydroxy position, by conventional methods (e.g. acylation, alkylsilylations).

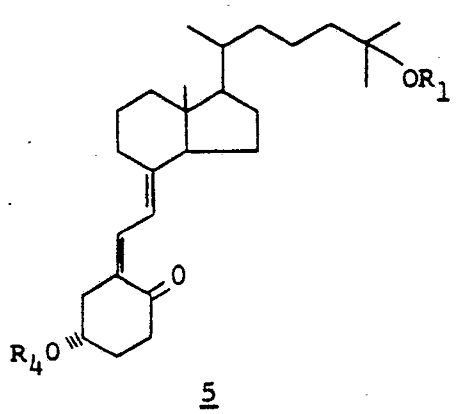

5

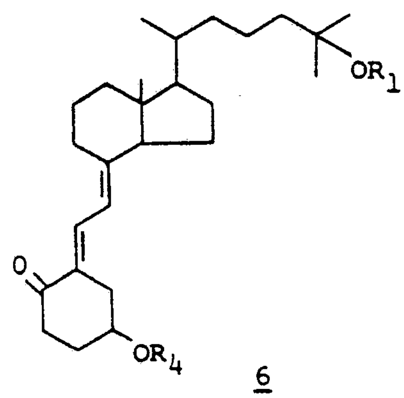

6

Thus $R_1$ and $R_4$ can independently represent hydrogen or a hydroxy-protecting group.

Alternatively, hydroxy-protected derivatives represented by structure *5* and *6*, bearing a hydroxy-protecting group at either C-3 C-25 or at both positions, can be obtained by subjecting the free hydroxy compounds to appropriate hydroxy-protecting procedures, which are well-known in the art. For example, acetylation of compound *5* ($R_1$, $R_4$ = H) with acetic anhydride in pyridine at room temperature gives the 3-monoacetate (*5*, $R_1$ = H, $R_4$ = acetyl) whereas the same reaction at elevated temperatures (75—100°) provides the 3,25-diacetate (*5*, $R_1$, $R_4$ = acetyl). Benzoylation or trimethylsilylation or the 3-monoacetate gives the 3-acetate-25-benzoate (*5*, $R_1$ = benzoyl, $R_4$ = acetyl) or the 3-acetate-25-trimethylsilyl derivative ($R_1$ = trimethylsilyl, $R_4$ = acetyl) respectively. The 3,25-diacetate can be selectively hydrolyzed (e.g. 5% KOH, for 10—30 min) to give the 25-monoacetate (*5*, $R_1$ = acetyl, $R_4$ = H), and this derivative can be reprotected at C-3 to give the 3,25-di-protected compound, where the protecting group may be the same or different. Exactly analogous procedures, applied to compounds of structure *6* or to the precursor compounds of structure *3* or *4*, provide the corresponding partially or fully hydroxy-protected derivatives already mentioned.

The 10-oxo-analogs of this invention, i.e. structures *5* and *6* ($R_1$ and $R_4$ = H), have been found to be highly effective ligands for binding to the cytosolic intestinal D-receptor protein, and since high affinity for this receptor protein is a characteristic of the biologically potent vitamin D metabolites and analogs (e.g. see DeLuca *et al.* Topics in Current Chemistry, vol. *83*, p. 1 (1979)), the novel compounds of this invention should find utility as therapeutically effective agents. The 25-hydroxy-substituted cholesterol-side chain in products *5* and *6* is a key structural element accounting for their high receptor-affinity, and it is this feature that distinguishes the compounds of this invention from the known 10-oxo-vitamin $D_2$ compounds, 5,6-*cis* and 5,6-*trans*-10-oxo-19-norvitamin $D_2$ disclosed in J. Chem. Soc., p. 837 and 843 (1958).

The following Examples further illustrate the present invention. The compound numbering corresponds to that given above.

Example 1

*7,8,25-Trihydroxy-7,8-dihydrovitamin $D_3$ 3-acetate* (compound *2*, $R_1$, $R_2$, $R_3$ = H; $R_4$ = acetyl)

To a solution of 250 mg of 25-hydroxyvitamin $D_3$ 3-acetate in 2.0 ml of dry pyridine was added 1.67 mL of a 10% solution of $OsO_4$ in pyridine. After 15 min all the starting material had been consumed and 10 mL of 10% $NaHSO_3$ was added. This solution was stirred for 30 min at room temperature, then diluted with 50 mL of 10% $NaHCO_3$ and extracted with ether (3 × 25 mL). The ether extracts were washed with water (2 × 25 mL), 1 N HCl (2 × 25 mL), sat. $NaHCO_3$ (2 × 25 mL), water (1 × 50 mL), dried over $Na_2SO_4$ and concentrated to an oil *in vacuo*. Preparative HPLC (high pressure liquid chromatography; 6.2 × 250 mm Zorbax-Sil column, 15% 2-propanol/hexanes) yielded 185 mg of compound *2* ($R_1$, $R_2$, $R_3$ = H; $R_4$ = acetyl) as an oil which eluted at 15 mL and possessed the following spectral characteristics: mass spectrum, m/e (relative intensity) 476 ($M^+$, 3), 458 (5), 416 (10), 298 (25), 245 (20), 136 (100), 59 (75); NMR δ 0.80 (3H, s, 18-$H_3$), 0.92 (3H, d, J = 6.0 Hz, 21-$H_3$), 1.23 (6H, s, 26-$H_3$ and 27-$H_3$), 2.04 (3H, s, 3-$OCOCH_3$), 4.81 (1H, m, 3-H), 4.91 (1H, d, J = 9.5 Hz, 7-H), 4.95 (1H, s, 19(Z)-H), 5.03 (1H, s, 19(E)-H), 5.58 (1H, d, J = 9.5 Hz, 6-H). Alkaline hydrolysis (5% KOH/MeOH, 30 min) of this product provides the corresponding tetrahydroxy-compound, structure *2*, $R_1$, $R_2$, $R_3$ and $R_4$ = H.

Example 2

(6*R*)-*25-Hydroxy-3,5-cyclovitamin $D_3$ 6-methyl ether*

A solution of 300 mg of 25-hydroxyvitamin $D_3$ and 350 mg of p-toluenesulfonyl chloride in 2.0 mL of dry pyridine was allowed to react for 48 hr at 5°C with stirring. The solution was then quenched with sat. $NaHCO_3$ and the aqueous phase extracted with ether (3 × 30 mL). The ether extracts were washed with 1 N HCl (2 × 20 mL), sat. $NaHCO_3$ (2 × 30 mL), $H_2O$ (1 × 50 mL), dried over $MgSO_4$ and concentrated *in vacuo*. The resulting crude 3β-tosylate derivative was taken up in 15.0 mL of anhydrous methanol containing 800 mg of $NaHCO_3$ and heated to 55°C for 6.0 hr. At the end of this period the reaction was cooled, concentrated to 5 mL, diluted with ether and washed with water (3 × 30 mL). After drying over $MgSO_4$ the ether solution was concentrated to an oil which was shown to be 80% 25-hydroxy-3,5-cyclovitamin $D_3$ by tlc (thin layer chromatography) analysis and therefore suitable for subsequent reactions.

Example 3

(6*R*)-*10,19-Dihydro-10,19-25-trihydroxy-3,5-cyclovitamin $D_3$ 6-methyl ether* (compound *1*, $R_1$, $R_2$, $R_3$ = H; X = $CH_3$)

To 462 mg of the product of Example 2 in 3.0 mL of dry pyridine was added 3.1 mL of a 10% $OsO_4$ solution in pyridine; the reaction was continued for 10 min, after which it was quenched with 15 mL 10% $NaHSO_3$. After 30 min the reaction was further diluted with 50 ml of $NaHSO_3$ and extracted with ether (3 × 30 mL). The organic extracts were washed with 1 N HCl (3.40 mL), water (2 × 40 mL), dried over $MgSO_4$ and concentrated to an oil *in vacuo*. After HPLC (6.2 × 250 mm, Zorbax-Sil, 15% 2-propanol/hexanes) compound *1* ($R_1$, $R_2$, $R_3$ = H; X = $CH_3$) was obtained in 70% yield as a colorless oil (16 mL elution volume in 70% yield): mass spectrum, m/e (relative intensity), 448 ($M^+$,3), 430 (5), 416 (45), 398 (15), 367 (40), 269 (40), 245 (35), 59 (100); NMR 0.32 (1H, m, 3-H), 0.52 (2H, m, 4-$H_2$), 0.56 (3H, s, 18-$H_3$), 0.90 (3H, d, J = 6.0, 21-$H_3$), 1.23 (6H, s, 26-$H_3$ and 27-$H_3$), 3.25 (3H, s, 6-$OCH_3$), 3.63 (2H, m, 19-$H_2$), 4.60 (1H, d, J = 9.2Hz, 6-H), 4.78 (1H, d, J = 9.2Hz, 7-H).

Example 4

(5*Z*)- *and* (5*E*)-*10,19,25-Trihydroxy-10,19-dihydrovitamin $D_3$ 3-acetate* (compounds *3* and *4*, $R_1$, $R_2$, $R_3$ = 4; $R_4$ = acetyl)

A solution of 300 mg of 10,19-diol-cyclovitamin compound *1* ($R_1$, $R_2$, $R_3$ = H; X = CH) in 3.0 mL of glacial acetic acid was heated to 55°C for 154 min and then quenched by adding dropwise to ice/saturated, $NaHCO_3$. The ether extraction was (3 × 25 mL) washed with $H_2O$ (2 × 30 mL), dried over $MgSO_4$ and concentrated *in vacuo*. The oily crude product, subjected to HPLC purification (6.2 × 250 mm, Zorbax-Sil, 8% 2-propanol/hexanes), gave the 5,6-cis (5*Z*)-compound of structure *3* ($R_1$, $R_2$, $R_3$ = H; $R_4$ = acetyl), eluting at 49 mL, in 48% yield: $UV_{max}$ 252 nm; mass spectrum, m/e (relative intensity) 476 ($M^+$, 5), 458 (20), 416 (35), 398 (25), 245 (30), 185 (60), 134 (100), 59 (60), NMR δ 0.55 (3H, s, 18-$H_3$), 0.96 (3H, d, J = 6.0Hz, 21-$H_3$), 1.23 (6H, s, 26-$H_3$ and 27-$H_3$), 2.05 (3H, s, 3-$OCOCH_3$), 3.72 (2H, m, 19-$H_2$), 4.74 (1H, m, 3-H), 5.82 (1H, d, J = 11.2Hz, 7-H), 6.63 (1H, d, j = 11.2Hz, 6-H), and the 5.6-trans (5*E*)-isomer *4* ($R_1$, $R_2$, $R_3$ = H; $R_4$ = acetyl),

eluting at 27 mL in 18% yield: UV$\rightarrow_{max}$ = 250 nm; mass spectrum, m/e (relative intensity) 476 ($M^+$, 2) 458 (6), 416 (30), 398 (30), 245 (25), 185 (40), 134 (100), 59 (80); NMR δ 0.46 (3H, s, 18-$H_3$), 0.98 (3H, d, J = 6.2Hz, 21-$H_3$), 1.22 (6H, s, 26-$H_3$ and 27-$H_3$), 2.03 (3H, s, 3-$OCOCH_3$), 3.67 (2H, q-AB, J = 11.0Hz, 19-$H_2$), 4.7 (1H, m, 3-H), 6.02 (1H, d, J = 15Hz, 7-H), 6.30 (1H, d, J = 15Hz, 6-H). Mild base hydrolysis of the 3-monoacetates thus obtained gives the corresponding hydroxyvitamin analogs *3* and *4* where $R_1$, $R_2$, $R_3$, and $R_4$ = H.

Example 5

(5*Z*)- *and* (5*E*)-*10-oxo-25-hydroxy-19-nor-vitamin* $D_3$ (compounds *5* and *6*, $R_1$, $R_4$ = H)

A solution of 50 mg of compound *3* ($R_1$, $R_2$, $R_3$ = H; $R_4$ = acetyl) in 1.5 mL of methanol was treated with 0.5 mL of a saturated solution of $NaIO_4$ in $H_2O$. The reaction was heated to 50°C for 2.5 hr, diluted with $H_2O$ and extracted with ether (3 × 30 mL). The ether extracts were washed with $H_2O$ (2 × 20 mL), dried over $MgSO_4$ and concentrated *in vacuo* to give compound *5* ($R_1$ = H; $R_4$ = acetyl). This material was taken up in 3.0 mL of ethanol and treated with 1.0 mL of 5% methanolic NaOH for 30 min at room temperature. The reaction mixture was neutralized with 1 N HCl, concentrated *in vacuo* and diluted with ether (50 mL). The organic phase was washed with $H_2O$ (2 × 20 mL), dried over $MgSO_4$ and taken to an oil *in vacuo* which was purified via HPLC (6.2 × 250 mm Zorbax-Sil, 14% 2-propanol/hexanes) to give compound *5* ($R_1$, $R_4$ = H), eluting at 37 mL, in 72% yield: UV$\rightarrow_{max}$ 310 nm ( = 15,000); mass spectrum, m/e (relative intensity) 402 ($M^+$, 35), 384 (30), 369 (10), 359 (45), 341 (15), 273 (35), 177 (50), 135 (70), 133 (100), 59 (60); NMR δ 0.55 (3H, s, 18-$H_3$), 0.96 (3H, d, J = 6.0Hz, 21-$H_3$), 1.22 (6H, s, 26-$H_3$ and 27-$H_3$), 4.2 (1H, M, 3-H), 5.87 (1H, d, J = 12.6Hz, 6-H), 7.61 (1H, d, J = 12.6Hz, 7-H).

Similar treatment of the (5*E*)-triol 3-acetate (compound *4*, $R_1$, $R_2$, $R_3$ = H; $R_4$ = acetyl) afforded first the 10-oxo-3-acetate compound *6* ($R_1$ = H; $R_4$ = acetyl) and, after hydrolysis, the 5(*E*)-10-oxo analog *6* ($R_1$, $R_4$ = H) which on HPLC eluted at 34 mL in 14% 2-propanol/hexane and exhibited the following physical characteristics: UV$\rightarrow_{max}$ 307 ( = 24,000); mass spectrum, m/e (relative intensity) 402 ($M^+$, 30), 384 (30), 369 (20), 359 (40), 273 (40), 177 (60), 135 (40), 133 (100), 59 (40); NMR δ 0.56 (3H, s, 18-$H_3$), 0.95 (3H, d, J = 6.0Hz, 21-$H_3$), 1.23 (6H, s, 26-$H_3$ and 27-$H_3$), 4.2 (1H, M, 3-H), 6.65 (2H, g-AB, J = 11.8Hz, 6-H and 7-H).

**Claims**

1. A compound having the formula

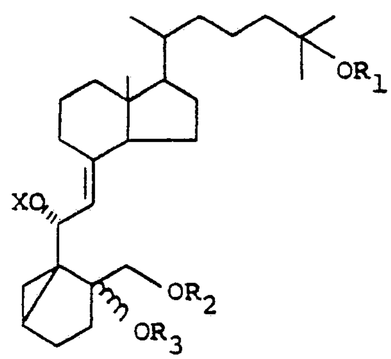

where each of $R_1$, $R_2$ and $R_3$, which may be the same or different, represents hydrogen or a hydroxy-protecting group, and X represents alkyl.

2. The compound of claim 1 wherein $R_1$, $R_2$ and $R_3$ are hydrogen, and X is methyl.

3. A compound having the formula:

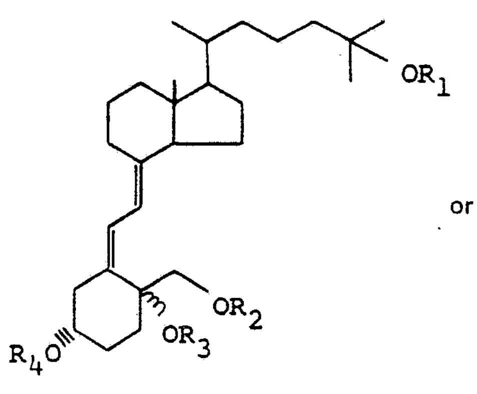

or

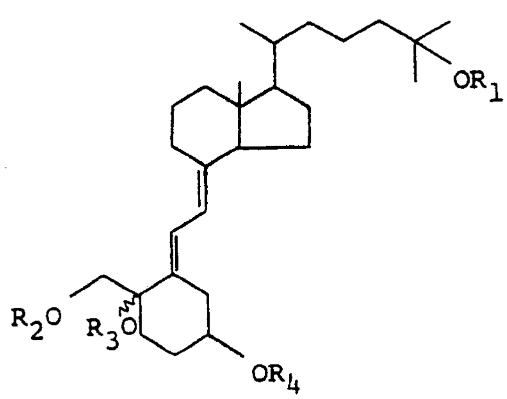

wherein $R_1$, $R_2$, $R_3$ and $R_4$, which may be the same or different, represent hydrogen or a hydroxy-protecting group.

4. A compound according to claim 3, wherein $R_1$, $R_2$, $R_3$ and $R_4$ represent hydrogen.

5. A compound according to claim 3, wherein $R_1$, $R_2$, and $R_3$ represent hydrogen, and $R_4$ is acyl.

6. A compound having the formula:

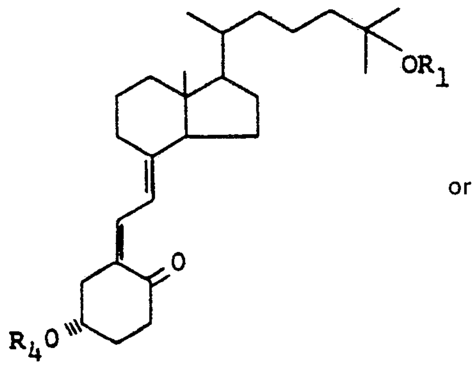

or

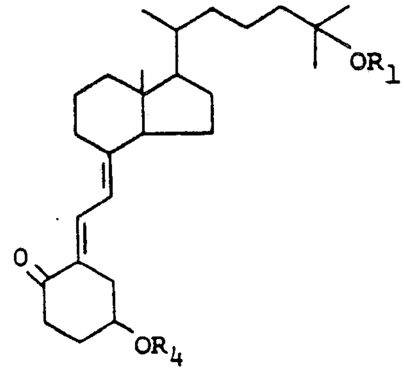

wherein $R_1$ and $R_4$, which may be the same or different represent hydrogen or a hydroxy-protecting group.

7. 25-Hydroxy-10-oxo-19-norvitamin $D_3$.

8. 25-Hydroxy-10-oxo-19-nor-5,6-*trans*-vitamin $D_3$.

9. A compound according to claims 1, 3 and 6 wherein at least one of $R_1$, $R_2$, $R_3$ ad $R_4$ represents hydrogen, acetyl, benzoyl or trimethylsilyl.

**Patentansprüche**

1. Verbindung der Formel

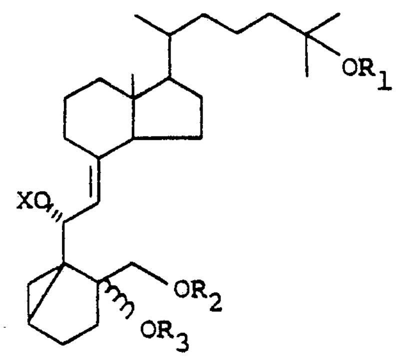

worin jeder der Reste $R_1$, $R_2$ und $R_3$, die gleich oder unterschiedlich sein können, für Wasserstoff oder eine Hydroxylschutzgruppe stehen, und X einen Alkylrest bedeutet.

2. Verbindung nach Anspruch 1, worin $R_1$, $R_2$ und $R_3$ für Wasserstoff stehen, und X eine Methylgruppe bedeutet.

3. Verbindung der Formel

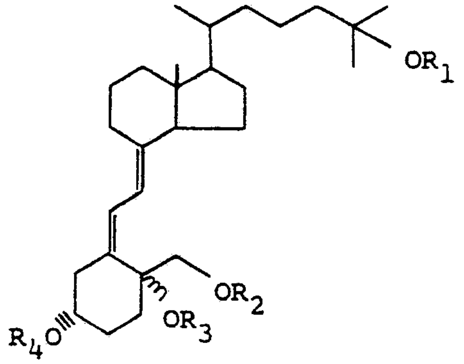

oder

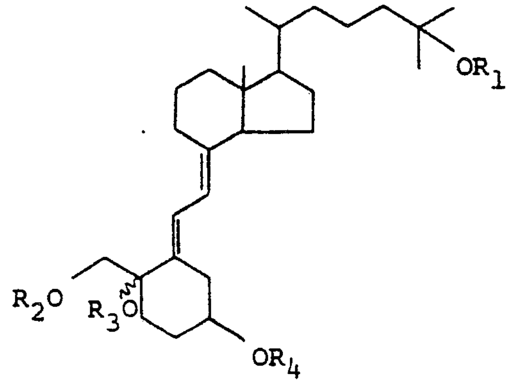

worin $R_1$, $R_2$, $R_3$ und $R_4$, die gleich oder unterschiedlich sein können, für ein Wasserstoffatom oder eine Hydroxylschutzgruppe stehen.

4. Verbindung nach Anspruch 3, worin $R_1$, $R_2$, $R_3$ und $R_4$ ein Wasserstoffatom darstellen.

5. Verbindung nach Anspruch 3, worin $R_1$, $R_2$ und $R_3$ ein Wasserstoffatom darstellen und $R_4$ eine Azylgruppe bedeutet.

6. Verbindung der Formel

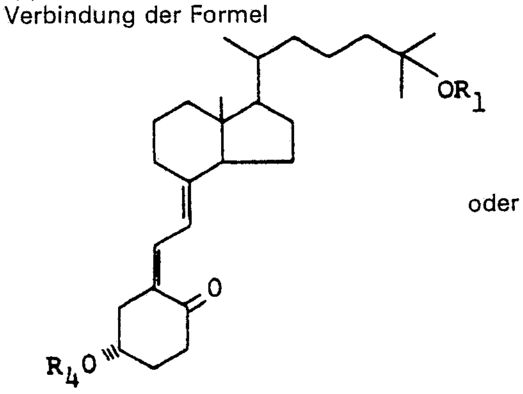

oder

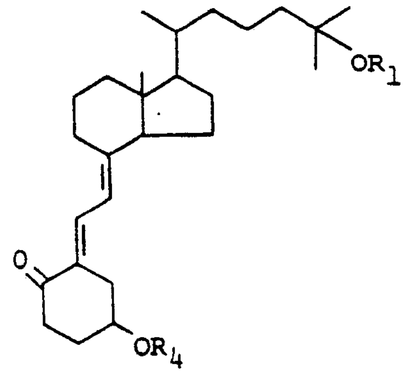

worin $R_1$ und $R_4$, die gleich oder unterschiedlich sein können, ein Wasserstoffatom oder eine Hydroxylschutzgruppe bedeuten.

7. 25-Hydroxy-10-oxo-19-norvitamin $D_3$.

8. 25-Hydroxy-10-oxo-19-nor-5,6-trans-Vitamin $D_3$.

9. Verbindung nach Anspruch 1, 3 und 6, worin mindestens einer der Reste $R_1$, $R_2$, $R_3$ und $R_4$ ein Wasserstoffatom, eine Acetylgruppe, eine Benzoylgruppe oder eine Trimethylsilylgruppe darstellt.

**Revendications**

1. Composé de formule

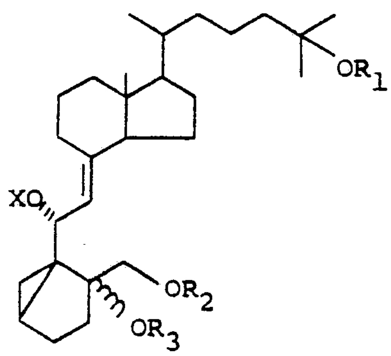

dans laquelle chacun de $R_1$, $R_2$ et $R_3$, qui peuvent être identiques ou différents, représente un hydrogène ou un groupe hydroxy-protecteur, et X représente un groupe alkyle.

2. Composé selon la revendication 1, dans lequel $R_1$, $R_2$ et $R_3$ sont des atomes d'hydrogène et X est un groupe méthyle.

3. Composé de formule:

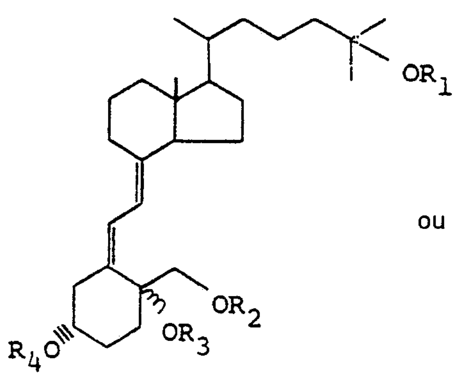

ou

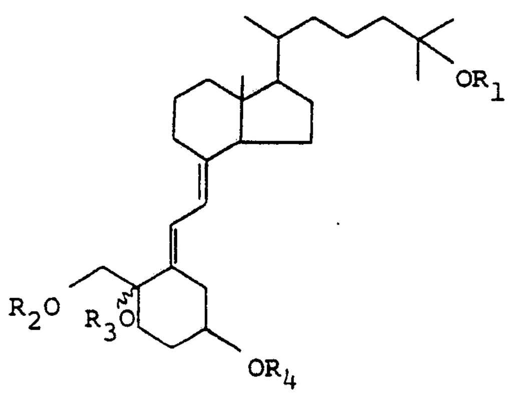

dans laquelle $R_1$, $R_2$, $R_3$ et $R_4$, qui peuvent être identiques ou différents, représentent un hydrogène ou un groupe hydroxy-protecteur.

4. Composé selon la revendication 3, dans lequel $R_1$, $R_2$, $R_3$ et $R_4$ représentent des atomes d'hydrogène.

5. Composé selon la revendications 3, dans lequel $R_1$, $R_2$ et $R_3$ représentent des atomes d'hydrogène, et $R_4$ est un groupe acyle.

6. Composé de formule:

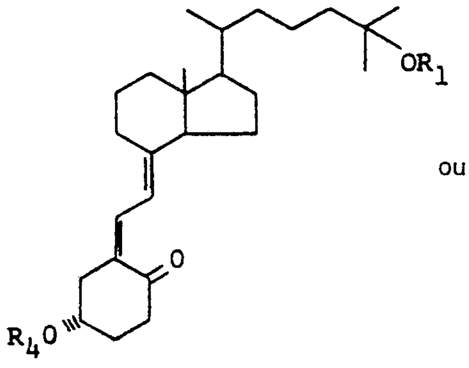

ou

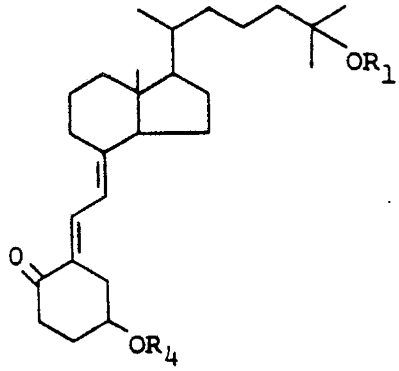

dans laquelle $R_1$ et $R_4$, qui peuvent être identiques ou différents, représentent un hydrogène ou un groupe hydroxy-protecteur.

7. 25-Hydroxy-10-oxo-19-norvitamine $D_3$.

8. 25-Hydroxy-10-oxo-19-nor-5,6-trans-vitamine $D_3$.

9. Composé selon les revendications 1, 3 et 6, dans lequel l'un au moins de $R_1$, $R_2$, $R_3$ et $R_4$ représente un hydrogène, un groupe acétyle, benzoyle ou triméthylsilyle.